# EUROPÄISCHE PATENTSCHRIFT

(11) **EP 0 992 217 B1**
(45) Veröffentlichungstag und Bekanntmachung des Hinweises auf die Patenterteilung: **26.07.2006**
(21) Anmeldenummer: 99120161.7
(22) Anmeldetag: 08.10.1999
(51) Int. Cl.: A61B 6/04

(54) **Operationstischsystem**
Operating table system
Système de table d'opération

(30) Priorität: 09.10.1998 DE 29818100 U
(43) Veröffentlichungstag der Anmeldung: 12.04.2000
(73) Patentinhaber: MAQUET GmbH & Co. KG, 76437 Rastatt (DE)
(72) Erfinder: Ballhaus, Heribert Dr.Ing., 90562 Heroldsberg (DE); Olszewski, Jan Donat, 76189 Karlsruhe (DE); Sutter, Klaus, 76437 Rastatt (DE)
(74) Vertreter: Schaumburg, Thoenes, Thurn, Landskron

(56) Entgegenhaltungen:
- GB-A- 2 057 830
- US-A- 3 584 321
- US-A- 4 105 923
- US-A- 4 131 802
- US-A- 4 641 823

## Beschreibung

Die Erfindung betrifft einen Operationstisch gemäß dem Oberbegriff des Anspruchs 1, ein solcher Operationstisch ist beispielsweise aus der EP-B1 0 691 119 bekannt.

Die GB-A 2 057 830 zeigt einen Operationstisch, dessen Patientenliegefläche nahe einem Längsende mit einer Säule verbunden ist und eine Mehrzahl von relativ zueinander verschwenkbaren Abschnitten umfaßt. Jeder Abschnitt hat einen Rahmen mit einem darauf befestigten Polster. Der Rahmen ist so ausgebildet, daß unter das Polster eine Kassette mit einem Röntgenfilm eingelegt werden kann.

Der Erfindung liegt die Aufgabe zugrunde, einen Operationstisch der eingangs genannten Art anzugeben, der es ermöglicht, einen auf dem Opertionstisch liegenden Patienten durchleuchten zu können.

Diese Aufgabe wird durch die im kennzeichnenden Teil des Anspruchs 1 angegebene Merkmale gelöst.

Mit der erfindungsgemäßen Lösung kann der Raum unterhalb der Patientenlagerfläche abschnittsweise völlig frei gemacht werden, um ein Röntgengerät unter den jeweiligen Abschnitt der Patientenliegefläche zu schieben. Auch besteht damit die Möglichkeit, unterschiedliche Abschnitte der Patientenliegefläche zu unterstützen, wenn die anderen Abschnitte zur Erzielung bestimmter Körperstellungen des Patienten verschwenkt werden sollen.

Die Erfindung betrifft femer ein Operationstischsystem für die CT-unterstützte Operation von Patienten.

Das Erstellen von Computertomographien zu Diagnosezwecken und zur Vorbereitung von Operationen ist bekannt. Hierzu wird der Patient üblicherweise auf eine Tischeinheit gelegt, die vor dem Computertomographen angeordnet ist und deren Tischplatte in den Computertomographen eingeschoben werden kann. Soll der Patient anschließend operiert werden, muß er von diesem Tisch auf die Liegefläche des Operationstisches umgebettet werden. Mit einer solchen Vorgehensweise ist es nicht möglich, während einer Operation eine ergänzende Computertomographie zu erstellen, da der Patient während der Operation in der Regel nicht bewegt, jedenfalls nicht von der Liegefläche des Operationstisches auf eine andere Unterlage umgelagert werden kann.

Aus der US-A 4 105 923 ist ein System bekannt, bei dem einem Computertomographen ein Stützgestell mit einer Koppelstation zugeordnet ist, die es ermöglicht, eine Lagerplatte zur Aufnahme eines Patienten von einem fahrbaren Gestell aus durch den Computertomographen zu ziehen, so daß ein entsprechendes Computertomogramm von dem Patienten erstellt werden kann. Die Lagerplatte ist Teil eines Bettes oder einer fahrbaren Transportliege für Patienten. Vor dem Computertomographen ist eine Hubeinrichtung angeordnet, mit deren Hilfe die fahrbare Transportliege in eine Übergabeposition angehoben werden kann, in der die Lagerplatte von der Transportliege an die Koppelstation übergebbar ist. Die intraoperative Erstellung eines Computertomogramms ist damit nicht möglich.

Der Erfindung liegt die Aufgabe zugrunde, ein Operationstischsystem der eingangs genannten Art anzugeben, das es ermöglicht, einen Patienten während einer Operation in einem Computertomographen zu untersuchen, der in unmittelbarer Nähe des Operationsraumes angeordnet sein kann.

Diese Aufgabe wird durch die im Anspruch 5 angegebenen Merkmale gelöst.

Bei der erfindungsgemäßen Lösung kann der Patient von dem Operationstisch in den Computertomographen eingeschoben werden, ohne daß er auf der Lagerplatte bewegt zu werden braucht. Dadurch ist es möglich, während einer Operation beispielsweise eine Kontrollaufnahme im Computertomographen zu machen und anschließend die Operation fortzusetzen, ohne daß der Patient von der Operationstisch-Liegefläche auf eine andere Unterlage umgelagert werden muß.

Vorzugsweise hat die Koppelstation einen Antrieb zum Verschieben der Lagerplatte von dem Untergestell auf das Stützgestell und vice versa, wobei dieser Antrieb auch dazu verwendet werden kann, den Patienten innerhalb des Computertomographen zu verschieben.
Der Antrieb kann beispielsweise Antriebsrollen oder -räder haben, welche die Lagerplatte formschlüssig oder kraftschlüssig erfassen. So kann der Antrieb mindestens ein über einen Motor antreibbares Zahnrad umfassen, das zum Eingriff mit einer an der Patienten-Lagerplatte vorgesehenen Zahnstange bestimmt ist.

Die Patienten-Liegefläche kann in an sich bekannter Weise in mehrere Abschnitte unterteilt sein, die um horizontale Achsen schwenkbar miteinander verbunden sind. Dabei sind die gegeneinander verschwenkbaren Abschnitte der Lagerplatte vorzugsweise über Filmscharniere miteinander verbunden, so daß keine die Röntgenfähigkeit beeinträchtigenden Metall-Scharnierteile erforderlich sind.

Um den Patienten möglichst schnell, erschütterungsfrei und präzise an die Koppelstation heranschieben zu können, ist es zweckmäßig, wenn das Untergestell auf einer an die Koppelstation anschließenden Führungsbahn verschiebbar ist.

Bei einer bevorzugten Ausführungsform der Erfindung hat der Rahmen des Operationstisches zwei Längsholme, an denen erste Führungsschienen für die Patienten-Lagerplatte und zweite Führungsschienen für mit den Stützsäulen verbundene Querholme ausgebildet sind. Das Untergestell bzw. die einzelnen Stützsäulen können höhenverstellbar sein.

Weitere Merkmale und Vorteile der Erfindung ergeben sich aus der folgenden Beschreibung, welche in Verbindung mit den beigefügten Zeichnungen die Erfindung anhand eines Ausführungsbeispieles erläutern. Es zeigen:
- Fig. 1: eine schematische Seitenansicht des erfindungsgemäßen Operationstischsystems mit einem Computertomographen,
- Fig. 2: eine Endansicht des in Fig. 1 dargestellten Tischsystems in Richtung des Pfeiles A in Fig. 1, und
- Fig. 3 bis 6: der Fig. 1 entsprechende Seitenansichten des erfindungsgemäßen Operationstischsystems in verschiedenen Phasen bei der Übergabe der Patienten-Lagerplatte von dem Operationstisch auf das Stützgestell innerhalb des Computertomographen.

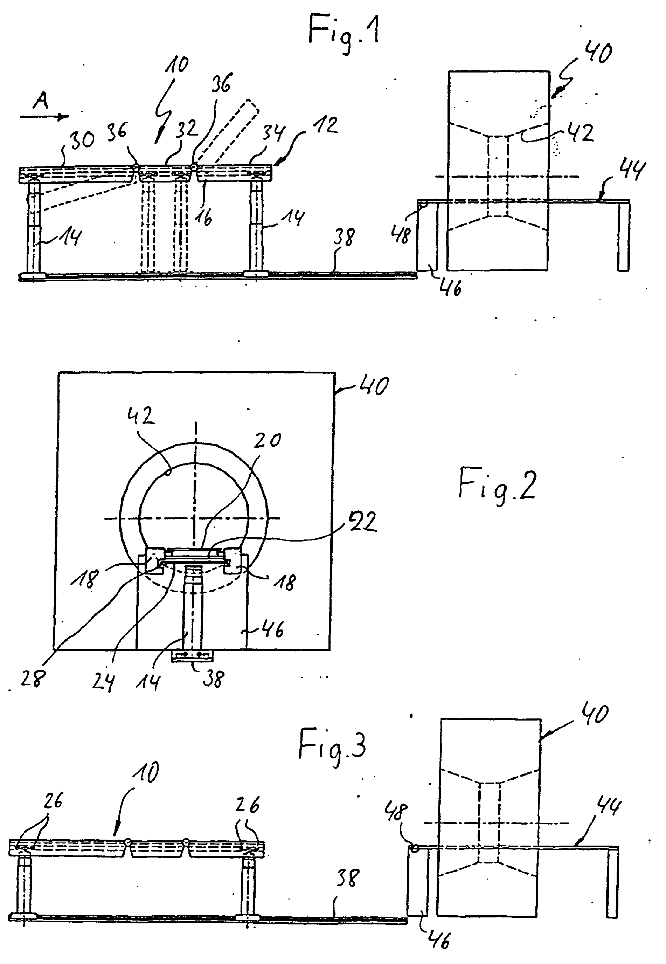

In Fig. 1 erkennt man einen allgemein mit 10 bezeichneten Operationstisch mit einer Patientenliegefläche 12, die auf zwei das Untergestell bildenden Stützsäulen 14 ruht. Die Patientenliegefläche 12 umfaßt einen Rahmen 16 mit zwei Längsholmen 18, die durch eine Rahmenplatte 22 aus einem röntgenstrahlendurchlässigen Material, z.B. Hartpapier miteinander verbunden sind. Auf der Rahmenplatte 22 liegt eine Patienten-Lagerplatte 20 auf, die auf Gleitführungen gegenüber der Rahmenplatte in Längsrichtung verschiebbar ist. Am oberen Ende jeder Stützsäule 14 ist ein Querholm 24 befestigt, der an seinem freien Ende Rollen 26 trägt, mit denen er in Nuten 28 in den Längsholmen 18 verschiebbar geführt ist. Die Rollen 26 können in den Nuten 28 gesperrt werden, so daß die Säulen 14 wahlweise und einzeln mit der Patientenliegefläche 12 starr verbunden werden können oder gegenüber dieser verschiebbar sind.

Die Längsholme 18 und die Rahmenplatte 22 sind in dem dargestellten Ausführungsbeispiel in drei Abschnitte 30, 32 und 34 unterteilt, die durch Gelenke 36 miteinander verbunden sind, so daß sie gegeneinander um horizontale Schwenkachsen verschwenkt werden können, wie dies in Fig. 1 durch die gestrichelt eingezeichneten Stellungen der Abschnitte 30 und 34 angedeutet ist. Die Lagerplatte 20 hat an den den Gelenken 36 entsprechenden Stellen ebenfalls jeweils ein Gelenk, so daß sie der Schwenkbewegung der Abschnitte 30 und 34 folgen kann. Da die Lagerplatte 20 für Röntgenstrahlen durchlässig sein soll, besteht sie vorzugsweise aus Kunststoff. Um die Röntgenfähigkeit nicht zu beeinträchtigen sind die entsprechenden Scharniere zwischen den Abschnitten der Lagerplatte 20 von Filmscharnieren oder anderen röntgenstrahlendurchlässigen Scharnieren gebildet.

Die Säulen 14 sind auf einer auf dem Boden befestigbaren oder in den Boden eingelassenen Führungsbahn 38 verschiebbar, so daß sie relativ zueinander verstellt werden können, wie dies in Fig. 1 durch die gestrichelt dargestellten Säulen wiedergegeben ist, welche den Mittelabschnitt 32 der Patientenliegefläche 12 unterstützen. Ferner kann der Operationstisch 10 als Ganzes auf dieser Führungsbahn 38 in Richtung auf einen Computertomographen 40 verstellt werden. Der Computertomograph 40 ist in herkömmlicher Weise ausgebildet und braucht nicht näher erläutert zu werden. Er hat eine zentrale Öffnung 42 durch die hindurch sich ein tischförmiges Stützgestell 44 erstreckt, das auf seiner dem Operationstisch 10 zugewandten Seite mit einer Koppelstation 46 verbunden ist. Das Stützgestell 44 dient zur Aufnahme der Lagerplatte 20, wobei die Koppelstation 46 einen durch eine Rolle 48 angedeuteten Antrieb enthält, um die Lagerplatte 20 von dem Operationstisch 10 herunterzuziehen und auf das Stützgestell 44 zu schieben. Dies kann beispielsweise dadurch geschehen, daß die Rolle 48 ein Zahnrad ist, das von einem nicht dargestellten Motor angetrieben wird und das in eine an der Lagerplatte 20 ausgebildete, nicht dargestellte Zahnstange eingreift.

Ebenso ist es denkbar, daß der Antrieb Reibrollen umfaßt, die die Lagerplatte 20 transportieren.

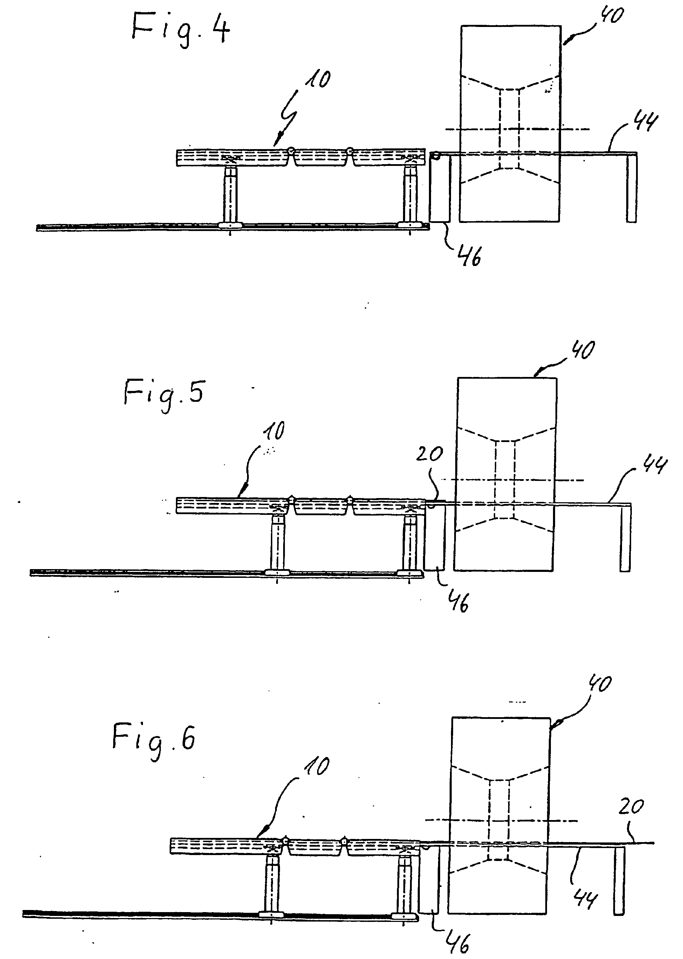

Die Übergabe erfolgt in der Weise, daß zunächst die Patientenliegefläche 12 aus der in Fig. 1 dargestellten Operationsstellung auf das in Fig. 3 dargestellte Niveau abgesenkt (oder bei Bedarf angehoben) wird, bei dem sich die Lagerplatte 20 auf einer dem Stützgestell 44 entsprechenden Höhe befindet (Fig. 3). Dann wird der Operationstisch 10 gemäß Fig. 4 an die Koppelstation 46 herangefahren und die Lagerplatte 20 gemäß Fig. 5 in Eingriff mit dem Antriebselement 48 der Koppelstation 46 gebracht. Nun kann die Lagerplatte zusammen mit dem darauf liegenden Patienten durch den Antrieb der Koppelstation 46 auf das Stützgestell 44 aufgeschoben werden und innerhalb des Computertomographen 40 jeweils in eine für die Erstellung der Aufnahme erforderliche korrekte Position bewegt werden.

Die vorstehend beschriebenen Vorgänge laufen bei der Übergabe der Lagerplatte 20 an den Operationstisch 10 in umgekehrter Reihenfolge ab. Wie man erkennt, besteht somit die Möglichkeit, selbst während einer laufenden Operation Kontrollaufnahmen in einem Computertomographen zu erstellen, ohne daß hierzu der Patient von der Operationstisch-Liegefläche auf eine Unterlage umgebettet werden muß, mit der er dann in den Computertomographen eingeschoben wird, wie dies bisher der Fall war.

Da die Patientenliegefläche 12 lösbar mit den Untergestell verbunden sein soll, besteht auch die Möglichkeit, den Operationstisch 10 und den Computertomographen 40 mit dem Stützgestell 44 in getrennten Räumen anzuordnen. Soll eine Kontrollaufnahme während einer Operation durchgeführt werden, kann die Operationstisch-Liegefläche in an sich bekannter Weise auf einen Wagen aufgesetzt und zu dem Computertomographen gefahren werden, wo dann die Übergabe der Lagerplatte an das Stützgestell 44 in der oben beschriebenen Weise erfolgt.

Das Stützgestell 44 kann höhenverstellbar sein und gegebenenfalls auch für eine bestimmte Patientenlagerung verstellbar, z.B. um eine Längs- oder Querachse schwenkbar sein. Das erfindungsgemäße Operationstischsystem ist auch in Kombination mit anderen Untersuchungsgeräten wie NMR-Geräten verwendbar.

## Patentansprüche

1. Operationstisch (10) mit einem Untergestell (14) und einer mit diesem lösbar verbundenen Patientenliegefläche (12), **dadurch gekennzeichnet, daß** die Patientenliegefläche (12) einen mit dem Untergestell verbundenen Rahmen (16) und eine in diesem längsverschiebbar geführte, für Röntgenstrahlen durchlässige Patientenlagenplatte hat, daß das Untergestell (14) auf einer Führungsbahn (38) motorisch oder manuell verschiebbar ist und mindestens zwei den Rahmen (16) tragende Stützsäulen hat, die auf der Führungsbahn (38) relativ zueinander verschiebbar sind, und daß der Rahmen (16) zwei Längsholme (18) hat, an denen erste Führungsschienen (22) für die Lagerplatte (20) und zweite Führungsschienen (28) für mit den Stützsäulen (14) verbundene Querholme (24) ausgebildet sind.

2. Operationstisch nach Anspruch 1, **dadurch gekennzeichnet, daß** die Patientenliegefläche (12) in mehrere Abschnitte (30, 32, 34) unterteilt ist, die um horizontale Achsen schwenkbar miteinander verbunden sind.

3. Operationstisch nach Anspruch 2, **dadurch gekennzeichnet, daß** die gegeneinander verschwenkbaren Abschnitte der Lagerplatte (20) über Röntgenstrahlen durchlässige Gelenke miteinander verbunden sind.

4. Operationstisch nach einem der Ansprüche 1 bis 3, **dadurch gekennzeichnet, daß** das Untergestell (14) höhenverstellbar ist.

5. Operationstischsystem für die CT-unterstützte Operation von Patienten, **gekennzeichnet durch** einen Operationstisch nach einem der Ansprüche 1 bis 4, ein zur Aufnahme der Lagerplatte (20) in einem Computertomographen (40) bestimmtes Stützgestell (44) und eine mit diesem verbundene Koppelstation zur Übergabe der Lagerplatte (20) von dem Operationstisch (10) auf das Stützgestell (44) und vice versa.

6. Operationstischsystem nach Anspruch 5, **dadurch gekennzeichnet, daß** die Koppelstation (46) einen Antrieb (48) zum Verschieben der Lagerplatte (20) von dem Untergestell (14) auf das Stützgestell (44) und vice versa hat.

7. Operationstischsystem nach Anspruch 6, **dadurch gekennzeichnet, daß** der Antrieb zum formschlüssigen Erfassen der Lagerplatte (20) ausgebildet ist.

8. Operationstischsystem nach Anspruch 7, **dadurch gekennzeichnet, daß** der Antrieb mindestens ein über einen Motor antreibbares Zahnrad umfaßt, das zum Eingriff mit einer an der Patientenlagerplatte (20) vorgesehenen Zahnstange bestimmt ist.

9. Operationstischsystem nach Anspruch 6, **dadurch gekennzeichnet, daß** der Antrieb zum kraftschlüssigen Transport der Lagerplatte (20) ausgebildet ist.

## Claims

1. An operating table (10) comprising an underframe (14) and a patient support surface (12) releasably connected with the underframe, **characterized in that** the patient support surface (12) has a frame (16) connected with the underframe and an X-ray permeable patient support plate longitudinally slidably guided in the frame, **in that** the underframe (14) is slidably guided on a guide track (38) either by a motor or manually and has at least two support columns supporting the frame (16), the support columns being movable relative to one another on the guide track (38), and **in that** the frame (16) has two longitudinal beams (18) on which first guide rails (22) for guiding the support plate (20) and second guide rails (28) for guiding cross beams (24) connected with the support columns (14) are formed.

2. The operating table according to claim 1, **characterized in that** the patient support surface (12) is divided into several sections (30, 32, 34) which are connected with one another for pivotal movement about horizontal axes.

3. The operating table according to claim 2, **characterized in that** the sections of the support plate (20) which are pivotal relative to one another are connected with one another by X-ray permeable joints.

4. The operating table according to one of the claims 1 to 3, **characterized in that** the underframe (14) is adjustable in height.

5. An operating table system for the computer tomography assisted operation on patients, **characterized by** an operating table according to one of the claims 1 to 4, a support frame (44) provided for receiving the support plate (20) in a computer tomograph (40) and a coupling station connected with the support frame and provided for the transfer of the support plate (20) from the operating table (10) to the support frame (44) and vice versa.

6. The operating table system according to claim 5, **characterized in that** the coupling station (46) has a drive (48) for shifting the support plate (20) from the underframe (14) to the support frame (44) and vice versa.

7. The operating table system according to claim 6, **characterized in that** the drive is formed for a positive engagement with the support plate (20).

8. The operating table system according to claim 7, **characterized in that** the drive comprises at least one gear drivable by a motor and used for engagement with a rack provided on the patient support plate (20).

9. The operating table system according to claim 6, **characterized in that** the drive is formed for a frictional transport of the support plate (20).

## Revendications

1. Table d'opération (10) comportant un châssis (14) et une surface porteuse pour patient (12) reliée de façon amovible audit châssis, **caractérisée en ce que** la surface porteuse pour patient (12) comprend un cadre (16) relié au châssis et une plaque de support pour patient guidée pour être mobile longitudinalement dans ledit cadre et transparente aux rayons X, **en ce que** le châssis (14) est mobile en translation, manuellement ou de façon motorisée, sur un rail-guide (38) et comporte au moins deux colonnes de support qui supportent le cadre (16) et qui sont mobiles en translation l'une par rapport à l'autre sur le rail-guide (38), et **en ce que** le cadre (16) comporte deux longerons (18) au niveau desquels sont conformées des premières glissières de guidage (22) destinées à la plaque de support (20) et des deuxièmes glissières de guidage (28) destinées à des traverses (24) reliées aux colonnes de support (14).

2. Table d'opération selon la revendication 1, **caractérisée en ce que** la surface porteuse pour patient (12) est divisée en plusieurs portions (30, 32, 34) qui sont reliées entre elles en pouvant pivoter autour des axes horizontales.

3. Table d'opération selon la revendication 2, **caractérisée en ce que** les portions de la plaque de support (20), qui sont pivotantes l'une par rapport à l'autre, sont reliées entre elles par des articulations transparentes aux rayons X.

4. Table d'opération selon l'une des revendications 1 à 3, **caractérisée en ce que** le châssis (14) est réglable en hauteur.

5. Système de table d'opération pour l'opération de patients, assistée par tomographie informatisée, **caractérisé par** une table d'opération selon l'une des revendications 1 à 4, un châssis de support (44) destiné à recevoir la plaque de support (20) dans un appareil de tomographie informatisée (40), et une station de couplage reliée audit châssis de support et permettant de transférer la plaque de support (20) depuis la table d'opération (10) sur le châssis de support (44), et vice versa.

6. Système de table d'opération selon la revendication 5, **caractérisé en ce que** la station de couplage (46) comprend un entraînement (48) destiné à déplacer en translation la plaque de support (20) depuis le châssis (14) sur le châssis de support (44), et vice versa.

7. Système de table d'opération selon la revendication 6, **caractérisé en ce que** l'entraînement est conformé pour saisir la plaque de support (20) par complémentarité de formes.

8. Système de table d'opération selon la revendication 7, **caractérisé en ce que** l'entraînement comporte au moins une roue dentée qui peut être entraînée par un moteur et qui est destinée à s'engager avec une crémaillère agencée sur la plaque de support pour patient (20).

9. Système de table d'opération selon la revendication 6, **caractérisé en ce que** l'entraînement est conformé pour transporter par friction la plaque de support (20).
